Europäisches Patentamt

European Patent Office

Office européen des brevets

(11) Veröffentlichungsnummer: **0 233 231**
**B1**

(12) EUROPÄISCHE PATENTSCHRIFT

(45) Veröffentlichungstag der Patentschrift: **20.06.90**

(51) Int. Cl.⁵: **A 61 K 9/48**, A 61 K 9/66

(21) Anmeldenummer: **86904825.6**

(22) Anmeldetag: **16.08.86**

(86) Internationale Anmeldenummer:
**PCT/EP86/00485**

(87) Internationale Veröffentlichungsnummer:
**WO 87/01034 26.02.87 Gazette 87/05**

(60) Teilanmeldung 89110787.2 eingereicht am
16/08/86.

(54) GELATINEKAPSELN UND VERFAHREN ZU IHRER HERSTELLUNG.

(30) Priorität: **20.08.85 DE 3529694**

(43) Veröffentlichungstag der Anmeldung:
**26.08.87 Patentblatt 87/35**

(45) Bekanntmachung des Hinweises auf die
Patenterteilung:
**20.06.90 Patentblatt 90/25**

(84) Benannte Vertragsstaaten:
**AT BE CH DE FR GB IT LI NL SE**

(56) Entgegenhaltungen:
EP-A-0 120 248
EP-A-0 199 034
FR-A-2 193 059
FR-A-2 261 752
US-A-2 580 683

(73) Patentinhaber: **R.P. Scherer GmbH
Gammelsbacher Strasse 2 Postfach 1243
D-6930 Eberbach/Baden (DE)**

(72) Erfinder: **FISCHER, Gerhard
Hohenstaufenstrasse 28/1
D-6930 Eberbach/Baden (DE)**
Erfinder: **SCHNEIDER, Benedikt
Melanchthonweg 50
D-7150 Backnang (DE)**
Erfinder: **JAHN, Helmut
Olgastrasse 109
D-7000 Stuttgart 1 (DE)**

(74) Vertreter: **Werner, Hans-Karsten, Dr. et al
Deichmannhaus am Hauptbahnhof
D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung sind Gelatinekapseln bestehend aus einer Kapselhülle und einer Kapselfüllung, wobei die Kapselhülle aus Gelatine, gegebenenfalls einem Weichmacher und weiteren Zusätzen besteht und die Kapselfüllung Wirkstoffe und/oder Diätmittel sowie gegebenenfalls weitere Zusätze und eine Flüssigkeit oder Pulver enthält. Weiterhin betrifft die Erfindung Verfahren zur Herstellung derartiger Gelatinekapseln.

Die Herstellung von Weichgelatinekapseln ist z.B. in Lachmann, Theory and Practice of Industrial Pharmacy, Lea and Febiger, Philadelphia, 2. Ausgabe, ausführlich beschrieben. Es wird dort ausgeführt, daß die Art der Weichkapselfüllgüter im wesentlichen auf drei Kategorien beschränkt ist, nämlich nicht wassermischbare, nicht flüchtige oder leicht flüchtige Träger wie Öle, Fette, etherische Öle, chlorierte Kohlenwasserstoffe, Ester, Ether, höhere Alkohole und organische Säuren. Diese Kategorie von Hilfs- und Wirkstoffen ist daher relativ einfach zu verkapseln und läßt einen ziemlich großen Spielraum für die Formulierung der Kapselhülle.

Die zweite Kategorie umfaßt mit Wasser mischbare, nicht flüchtige Stoffe wie Polyethylenglykole oder Emulgatoren. Diese Kategorie ist weitaus schwieriger zu verarbeiten. So beschreibt z.B. die DE-OS 33 07 353 ein Verfahren zur Herstellung von polyethylenglykolhaltigen Weichgelatinekapseln, wonach Polyethylenglykol nur dann zuverlässig verkapselt werden kann, wenn ganz bestimmte Parameter sowohl bezüglich der Kapselhülle als auch bezüglich des Kapselfüllgutes eingehalten werden.

Die Verkapselungsprobleme können damit erklärt werden, daß polyethylenglykolhaltige Füllungen in starke Wechselwirkungen mit der Kapselhülle treten und so die Lagerstabilität der Kapseln nicht mehr ohne weiteres gewährleistet ist.

Die dritte Kategorie schließlich umfaßt wassermischbare und relativ schwer flüchtige Komponenten wie z.B. Glycerin, Propylenglykol oder Benzylalkohol. Bei dieser Kategorie sind die in der Kategorie (2) beschriebenen Wechselwirkungen so stark, daß Komponenten der letzten Kategorie nur in Konzentrationen bis zu maximal 10% verkapselt werden können.

Als nicht verkapselbar gelten nach Lachmann, loc.cit. Flüssigkeiten mit mehr als 5% Wasser und niedermolekulare, organische, wasserlösliche Verbindungen, insbesondere niedere Alkohole, wie Ethylalkohol, Ketone und Amine.

Besondere probleme bereitet auch die Verarbeitung wasserlöslicher oder hygroskopischer Wirkstoffe in Gelatinekapseln, da diese ebenso die Kapselwand negativ beeinflussen.

Die US-A-2 580 683 beschreibt Gelatinekapseln, die mit hoch konzentrierten Lösungen von hygroskopischem Material, wie Glukosesirup, gefüllt sind, wobei die Kapselfüllung so hoch konzentriert an hygroskopischem Material ist, daß die Kapselfüllung nicht mehr in der Lage ist, die Kapselhülle zu zerstören. Weiterhin wird hierin beschrieben, daß die Konzentration an hygroskopischem Material der Kapselfüllung verringert werden kann, wenn in der Kapselhülle auf ein Teil Gelatine zusätzlich ein Teil Zucker eingearbeitet wird. Durch diese Maßnahme ist es möglich, etwas niedrigerviskose Kapselfüllungen zu verarbeiten. Auf alle Fälle muß jedoch eine sehr hohe Konzentration an hygroskopischem Material in der Kapselfüllung vorhanden sein, ohne daß dies durch konkrete Zahlenangaben festgelegt ist.

Die EP-A-0 120 248 beschreibt polyethylenglykolhaltige Weichgelatinekapseln und Verfahren zu ihrer Herstellung. Dabei werden übliche Kapselhüllen eingesetzt. Die Kapselfüllung besteht aus Lösungen oder Suspensionen des Wirkstoffes in Polyethylenglykol, von dem mindestens 50 Gew.-% ein mittleres Molekulargewicht von 600 aufweist. Zusätzlich enthält die Kapselfüllung Glycerin und/oder 1,2-Propylenglykol.

Aus der nicht vorpublizierten EP-A-0 199 034 ist bekannt die Verkapselung von Honig, Invertzucker oder Zuckersirup in normalen Weichgelatinehüllen, in dem der Kapselfüllung 2 bis 20 Gew.-% Glycerin beigemischt wurden. Während bei sonstigen Kapselfüllungen derartig hohe Mengen von Glycerin zur Zerstörung der Kapselhülle führen, wird dieses Glycerin offensichtlich durch Honig, Invertzucker oder Zuckersirup so an die Kapselfüllung gebunden, daß es keinen schädlichen Einfluß auf die Kapselhülle ausüben kann. Dennoch ist es nötig, die so hergestellten Weichgelatinekapseln geschützt zu lagern, da sie sonst nur einige Wochen haltbar sind.

Die Erfindung hat sich die Aufgabe gestellt, diese dritte, bisher nicht verkapselbare Gruppe von Substanzen zu verkapseln, d.h. Kapselfüllungen herzustellen, welche mindestens einen mit Wasser mischbaren, wasserlöslichen, wasserempfindlichen oder hydrophilen, leicht flüchtigen Stoff enthalten. Weiterhin hat sie sich die Aufgabe gestellt, nur schwierig verkapselbare Substanzen der zweiten Kategorie leichter zu verkapseln.

Es wurde überraschenderweise gefunden, daß diese Aufgabe dadurch gelöst werden kann, daß

a) die Kapselhülle als Zusatz mindestens 1 Gew.% eines Mittels enthält, welches in reiner Form mindestens 10 Gew.-% seines Eigengewichts an Wasser aufnehmen kann, ohne sich äußerlich zu verändern, wobei dieses Mittel aus einem oder mehreren Stoffen aus der Gruppe der Stärken, Stärkederivate, Zellulosen, Zellulosederivate, Milchpulver, nicht hygroskopischer Oligosaccharide, Magnesiumtrisilikat, Siliciumdioxid besteht und

b) die Kapselfüllung mindestens einen mit Wasser mischbaren, wasserlöslichen, wasserempfindlichen oder hydrophilen, leicht flüchtigen Stoff enthält.

Mit Hilfe dieser erfindungsgemäßen Zusätze zur Kapselhülle ist es erstmals möglich, Kapselfüllungen einzusetzen, die einen oder mehrere Stoffe enthalten aus der Gruppe der niederen Alkohole, Ether, Ester, Alkanale, Alkanone, Polyole und Amine. Weiterhin ist es erfindungsgemäß möglich, Kapselfüllungen herzustellen, welche einen oder mehrere Stoffe enthalten aus der Gruppe der konzentrierten wässrigen Zuckerlösungen, Stärkesirupe, hydrierten Stärkesirupe, Zuckerderivatlösungen und Honig. Schließlich ist es möglich, nur schwierig verkapselbare Substanzen, wie mit Wasser mischbare, nicht flüchtige Stoffe, wie Polyethylenglykole oder Emulgatoren problemlos zu verkapseln.

Von besonderer Bedeutung sind Weichgelatinekapseln, wie sie insbesondere im pharmazeutischen Sektor zum Einsatz kommen, und die unzerkaut geschluckt werden sollen. Erfindungsgemäß lassen sich aber auch weiche Kaukapseln herstellen, die insbesondere im Lebensmittel- und Diätbereich zum Einsatz kommen können. Prinzipiell ist es aber auch möglich, mit Hilfe der erfindungsgemäßen Zusätze zur Kapselhülle stabile Hartgelatinekapseln herzustellen, die trotz der ungewöhnlichen Kapselfüllungen lagerbeständig sind.

Gegenstand der vorliegenden Erfindung sind somit Gelatinekapseln bestehend aus

a) einer Kapselhülle und

b) einer Kapselfüllung, wobei die Kapselhülle aus Gelatine, gegebenenfalls einem Weichmacher und weiteren Zusätzen besteht und die Kapselfüllung Wirkstoffe und/oder Diätmittel sowie gegebenenfalls weitere Zusätze und eine Flüssigkeit oder Pulver enthält, dadurch gekennzeichnet, daß

a) die Kapselhülle als Zusatz mindestens 1 Gew.-% eines Mittels enthält, welches in reiner Form mindestens 10 Gew.-% seines Eigengewichts an Wasser aufnehmen kann, ohne sich äußerlich zu verändern, wobei dieses Mittel aus einem oder mehreren Stoffen aus der Gruppe der Stärken, Stärkederivate, Zellulosen, Zellulosederivate, Milchpulver, nicht hygroskopischer Oligosaccharide, Magnesiumtrisilikat, Siliciumdioxid besteht und

b) die Kapselfüllung mindestens einen mit Wasser mischbaren, wasserlöslichen, wasserempfindlichen oder hydrophilen, leicht flüchtigen Stoff enthält.

Ein weiterer Gegenstand der Erfindung ist das Verfahren zur Herstellung von derartigen Gelatinekapseln, dadurch gekennzeichnet, daß

a) dem Gelatinesud als Zusatz eine Suspension oder Lösung von mindestens 1 Gew.-% eines Mittels zugegeben wird, welches in reiner Form mindestens 10 Gew.-% seines Eigengewichts an Wasser aufnehmen kann ohne sich äußerlich zu verändern, wobei dieses Mittel aus einem oder mehreren Stoffen aus der Gruppe der Stärken, Stärkederivate, Zellulosen, Zellulosederivate, Milchpulver, nicht hygroskopischer Oligosaccharide, Magnesiumtrisilikat, Siliciumdioxid besteht und

b) als Kapselfüllung mindestens ein mit Wasser mischbarer, wasserlöslicher, wasserempfindlicher oder hydrophiler, leicht flüchtiger Stoff eingesetzt wird.

Es ist bisher noch völlig ungeklärt, wieso der erfindungsgemäße Zusatz von Mitteln, welche in reiner Form mindestens 10 Gew.-% ihres Eigengewichts an Wasser aufnehmen können, ohne sich äußerlich zu verändern, die Eigenschaften der Kapselhülle derartig verändert, daß bisher als nicht verkapselbar geltende Kapselfüllungen zum Einsatz kommen können.

Die Wahl und Konzentration dieses Mittels richten sich in gewissem Maße nach der speziellen Kapselfüllung. Ebenso ist die Art der Einarbeitung dieser Mittel in die Gelatinehülle bis zu einem gewissen Grade produktabhängig. Dennoch sprechen alle bisherigen Ergebnisse dafür, daß es allein durch den Zusatz dieser Mittel gelingt, die Kapselhülle gegen Einflüsse aus der Kapselfüllung und der Umwelt zu inertisieren und die Durchlässigkeit für alle wasserlöslichen, hydrophilen und leicht flüchtigen Stoffe zu verringern. Erstaunlicherweise können diese Mittel bei der Verarbeitung entweder als Dispersion oder sogar als Lösung dem Gelatinesud zugegeben werden, d.h. also in einer Form, in der sie aufgrund der großen Wassermenge durchaus in der Lage sind, sich äußerlich zu verändern. In der endgültigen Kapselhülle scheinen sie eine Art Pufferwirkung auf den Wassergehalt der Hülle und der Kapselfüllung auszuüben, ohne daß jedoch diese Wirkung eine Erklärung dafür liefern kann, warum hierdurch erfindungsgemäß stabile und brauchbare Kapseln entstehen.

Die erfindungsgemäßen Zusätze zur Kapselhülle können aus den verschiedensten Stoffen ausgewählt sein. Besonders bewährt haben sich zunächst Stärken und Stärkederivate sowie Zellulosen und Zellulosederivate. Hierbei handelt es sich somit um native Kartoffel-, Mais-, Weizen- oder Tapiokastärke. Als Stärkederivate kommen physikalisch behandelte, modifizierte Stärken, oxidierte, hydrolysierte, enzymbehandelte, vernetzte, veresterte oder veretherte Stärken in Frage. Als Zellulose ist insbesondere mikrokristalline Zellulose verwendbar sowie Zellulosederivate, welche die erfindungsgemäßen Bedingungen bezüglich des Verhaltens gegenüber Wasser erfüllen.

Erstaunlicherweise sind auch Milchpulver und nicht hygroskopische Oligosaccharide geeignet. Aber auch anorganische Stoffe wie Magnesiumtrisilikat und kolloidales Siliciumdioxid erfüllen die erfindungsgemäße Aufgabe.

Einige dieser Stoffe sind in der Lage, wesentlich mehr als 10 Gew.-% ihres Eigengewichts an Wasser aufzunehmen, ohne sich äußerlich zu verändern. Andere Mittel dieser Stoffgruppe werden von größeren Mengen Wasser sogar klar gelöst. Dennoch erfüllen sie die erfindungsgemäße Aufgabe bezüglich der Verbesserung der Kapselhülle.

Zur Durchführung des erfindungsgemäßen Verfahrens zur Herstellung von Gelatinekapseln werden die Mittel in Form einer Suspension oder Lösung dem Gelatinesud zugegeben. Nach der üblichen Füllung und Trocknung der Kapseln entstehen unmittelbar brauchbare Produkte, auch wenn sie mit bisher als nicht verkapselbar geltenden Kapselfüllungen gefüllt wurden.

Als besonders problematisch galt bisher beispielsweise Ethanol. Übliche Weichgelatinekap-

seln, die nach dem Stand der Technik hergestellt wurden, lassen Ethanol durch die Kapselwand unmittelbar nach der Trocknung diffundieren, so daß die Kapseln ständig an Gewicht und Form verlieren und nicht mehr völlig auftrocknen. Bei anderen, mit Wasser mischbaren, wasserlöslichen oder gegen Wasser empfindlichen Substanzen war es nach dem Stand der Technik nicht möglich, ein Diffundieren des Wassers in die Kapselfüllung bzw. ein Diffundieren der Kapselfüllung in die Kapselhülle zu vermeiden.

Beides führte zu qualitativ nicht akzeptablen Produkten. Erstaunlicherweise wird diese unerwünschte Wechselwirkung zwischen Kapselhülle und Kapselfüllung durch den Zusatz der oben beschriebenen Mittel so weit unterdrückt, daß stabile und lagerfähige Kapseln entstehen. Leicht wasserlösliche oder hygroskopische Arzneimittel haben beispielsweise der Gelatinehülle so viel des Restwassers entzogen, daß diese versprödeten. Bei Weichgelatinekapseln konnte man dieser Versprödung bis zu einem gewissen Grad dadurch Einhalt gebieten, daß der Gelatinehülle größere Mengen ausgewählter Weichmacher zugesetzt wurden. Bei Hartgelatinekapseln, die keinen Weichmacher in der Hülle enthalten, ist jedoch eine solche Kompensation nicht möglich. Durch den erfindungsgemäßen Zusatz der oben erwähnten Mittel ist es somit auch möglich, hygroskopische Arzneimittel stabil in Hartgelatinekapseln abzufüllen.

Bei Weichgelatinekapseln hingegen haben gut wasserlösliche Substanzen stets dazu geführt, daß sie aus der Kapselfüllung in die Kapselhülle diffundiert sind und damit die Stabilität und Qualität der Kapselhülle beeinträchtigt haben. Auch nach dem Auftrocknen der Kapsel kann dieser Diffusionsprozeß weiterlaufen, so daß es nach einiger Zeit zu völlig unakzeptabler Anreicherung der Wirkstoffe in der Gelatinehülle kommt. Dies hat dann dazu geführt, daß die Gelatinehülle nachträglich wieder weich wurde oder im Extremfall die Kapseln sogar an der Verschweißungsnaht aufgerissen sind. All diese unerwünschten Wechselwirkungen zwischen Kapselfüllung und Kapselhülle können überraschenderweise erfindungsgemäß beseitigt oder in so starkem Maße zurückgedrängt werden, daß sie nicht mehr stören.

Auch die Wechselwirkung zwischen Kapselhülle und Umwelt wird erfindungsgemäß erheblich zurückgedrängt. So sind Kapseln im allgemeinen nur bei 15 bis 25°C und einer relativen Luftfeuchte unter 60°C dauerhaft lagerfähig, da die Kapseln durch Aufnahme von Luftfeuchtigkeit weich werden und/oder beginnen zusammenzukleben. Die erfindungsgemäßen Kapseln sind weitaus weniger anfällig gegen Verkleben und Weichwerden, was durch eine verminderte Aufnahme von Wasser aus der Umgebung erklärt werden könnte.

In den nachfolgenden Beispielen sind typische Ausführungsformen der erfindungsgemäßen Gelatinekapseln sowie das Verfahren zu ihrer Herstellung näher erläutert.

## Beispiel 1

Aus 48 kg Gelatine, 16 kg Glycerin und 36 kg Wasser wurde in an sich bekannter Weise ein Gelatinesud hergestellt. Zu diesem Sud wurde eine homogene Verreibung aus 20 kg Maisstärke in 28 kg Glycerin zugefügt. Daraus wurden Gelatinekapseln hergestellt, die mit reinem Glycerin gefüllt waren.

Zum Vergleich wurde die identische Formulierung hergestellt, jedoch ohne Zusatz von Maisstärke. Die erfindungsgemäße Formulierung ließ sich einwandfrei verarbeiten, zeigte eine feste Gelatinehülle und war lagerstabil, während die Vergleichsformulierung bereits nach der Verarbeitung deformiert war, weich und klebrig blieb und nicht weiterverarbeitbar war.

## Beispiel 2

Die gleiche Rezeptur wie im Beispiel (1) wurde mit Propylencarbonat gefüllt. Zum Vergleich wurde wiederum eine Rezeptur ohne Zusatz von Maisstärke hergestellt. Wiederum zeigte sich, daß die erfindungsgemäßen Gelatinekapseln sich einwandfrei verarbeiten ließen, eine feste Gelatinehülle aufwiesen und lagerstabil waren, während das Vergleichsbeispiel unmittelbar nach der Verarbeitung bereits deformiert, weich und klebrig war und nicht weiterverarbeitet werden konnte.

## Beispiele 3 und 4

Die gleiche Rezeptur wie im Beispiel (1) wurde mit einer Mischung aus gleichen Teilen Polyethylenglykol 400 und Ethylalkohol sowie einer Mischung aus gleichen Teilen Propandiol und Ethylalkohol gefüllt.

Wiederum waren die erfindungsgemäßen Rezepturen einwandfrei verarbeitbar und gaben lagerstabile, feste Produkte, während die Vergleichsbeispiele ohne Zusatz von Maisstärke schon unmittelbar nach der Verarbeitung deformiert waren, weich und klebrig blieben und daher nicht weiter verarbeitbar waren.

## Beispiele 5 und 6

Aus 5 kg Zucker, 3,8 kg Glucosesirup, 0,4 kg einer 70%igen wässrigen Sorbitlösung, 6 kg dünnkochendem Kartoffelstärkeacetat und 5,8 kg Wasser wurde eine Lösung hergestellt und diese in 60 kg eines Gelatinesudes eingerührt, welcher aus 46% Gelatine, 19,8% Glycerin und 34,2% Wasser bestand.

In dieses Material für die Kapselhülle wurde eine 70%ige wässrige Sorbit/Sorbitanlösung bzw. eine Mischung aus 80% Glucosesirup, 10% Glycerin und 10% Wasser gefüllt. Es entstanden stabile, weiche Kaukapseln. Als Vergleich wurde der gleiche Gelatinesud ohne Kartoffelstärkeacetat und Zuckerzusatz hergestellt. Wiederum konnte festgestellt werden, daß die Gelatinehülle ohne Zuschlagstoffe nicht formstabil war.

## Beispiel 7

Aus 48 kg Gelatine, 16 kg Glycerin und 36 kg Wasser wurde wie im Beispiel (1) ein Gelatinesud hergestellt. Zu diesem Sud wurde eine Verreibung aus 28 kg Magermilchpulver, 48 kg Glycerin und 16 kg Wasser gegeben. Daraus wurden Kapseln hergestellt, die pro Kapsel eine Lösung von 250 mg Chloralhydrat in 173 mg Polyethylenglykol 400 enthielten.

Im Gegensatz zum Vergleichsbeispiel ohne Magermilchpulver war die Gelatinehülle noch so hart, daß bei leichtem Druck keine Verformung der Gelatinehülle eintrat.

### Beispiel 8

Aus 48 kg Gelatine, 16 kg Glycerin und 36 kg Wasser wurde wie im Beispiel (1) ein Gelatinesud hergestellt. Zu diesem wurde eine homogene Verreibung aus 10 kg mikrokristalliner Zellulose und 14 kg Glycerin zugefügt. Daraus wurden Weichgelatinekapseln hergestellt, die im Füllgut 85% Honig und 15% Glycerin enthielten. Es entstanden feste und lagerstabile Gelatinekapseln, die qualitativ nicht zu beanstanden waren.

### Beispiel 9

Aus 33 kg Gelatine und 61 kg Glycerin wird ein Gelatinesud hergestellt. Diesem Sud wird eine Lösung von 5 kg Stärkeacetat in 5 kg Wasser zugesetzt. Dieser Gelatinesud wird in bekannter Weise zu Hartgelatinekapseln weiterverarbeitet. In diese Kapseln wird eine Mischung aus 60% Cholinchlorid, 10% Lactose, 19% Maisstärke und 1% Magnesiumstearat gefüllt. Im Gegensatz zum Vergleichsbeispiel ohne Stärkeacetat versprödet die Kapselhülle nicht.

### Patentansprüche

1. Gelatinekapseln bestehend aus
a) einer Kapselhülle und
b) einer Kapselfüllung, wobei die Kapselhülle aus Gelatine, gegebenenfalls einem Weichmacher und weiteren Zusätzen besteht und die Kapselfüllung Wirkstoffe und/oder Diätmittel sowie gegebenenfalls weitere Zusätze und eine Flüssigkeit oder Pulver enthält, dadurch gekennzeichnet, daß
a) die Kapselhülle als Zusatz mindestens 1 Gew.-% eines Mittels enthält, welches in reiner Form mindestens 10 Gew.-% seines Eigengewichts an Wasser aufnehmen kann, ohne sich äußerlich zu verändern, wobei dieses Mittel aus einem oder mehreren Stoffen aus der Gruppe der Stärken, Stärkederivate, Zellulosen, Zellulosederivate, Milchpulver, nicht hygroskopischer Oligosaccharide, Magnesiumtrisilikat, Siliciumdioxid besteht und
b) die Kapselfüllung mindestens einen mit Wasser mischbaren, wasserlöslichen, wasserempfindlichen oder hydrophilen, leicht flüchtigen Stoff enthält.

2. Gelatinekapseln gemäß Anspruch 1, dadurch gekennzeichnet, daß die Kapselfüllung einen oder mehrere Stoffe enthält aus der Gruppe der niederen Alkohole, Ether, Ester, Alkanale, Alkanone, Polyole und Amine.

3. Gelatinekapseln gemäß Anspruch 1, dadurch gekennzeichnet, daß die Kapselfüllung einen oder mehrere Stoffe enthält aus der Gruppe der konzentrierten wäßrigen Zuckerlösungen, Stärkesirupe, hydrierten Stärkesirupe, Zuckerderivatlösungen und Honig.

4. Gelatinekapseln gemäß einem der Ansprüche 1 bis 3, dadurch gekennzeichnet, daß die Kapselhülle die einer Weichgelatinekapsel ist.

5. Gelatinekapseln gemäß einem der Ansprüche 1 bis 3, dadurch gekennzeichnet, daß die Kapselhülle die einer weichen Kaukapsel ist.

6. Verfahren zur Herstellung von Gelatinekapseln gemß einem der Ansprüche 1 bis 5, dadurch gekennzeichnet, daß
a) dem Gelatinesud als Zusatz eine Suspension oder, Lösung von mindestens 1 Gew.-% eines Mittels zugegeben wird, welches in reiner Form mindestens 10 Gew.-% seines Eigengewichts an Wasser aufnehmen kann, ohne sich äußerlich zu verändern, wobei dieses Mittel aus einem oder mehreren Stoffen aus der Gruppe der Stärken, Stärkederivate, Zellulosen, Zellulosederivate, Milchpulver, nicht hygroskopischer Oligosaccharide, Magnesiumtrisilikat, Siliciumdioxid besteht und
b) als Kapselfüllung mindestens ein mit Wasser mischbarer, wasserlöslicher, wasserempfindlicher oder hydrophiler, leicht flüchtiger Stoff eingesetzt wird.

### Revendications

1. Capsules de gélatine constituées par
a) une enveloppe de capsule et
b) un remplissage de capsule dont l'enveloppe est en gélatine avec, le cas échéant, un émollient et d'autres additions et dont le remplissage contient des substances actives et/ou des produits diététiques ainsi que, le cas échéant, d'autres additions et un liquide ou une poudre, caractérisée en ce que
a) l'enveloppe de la capsule contient comme addition 1% de poids d'un produit qui, sous sa forme pure, peut absorber au moins 10% de son propre poids d'eau, sans subir de modifications extérieures, ce produit étant constitué par une ou plusieurs matières choisies dans le groupe des amidons, des dérivés d'amidon, des celluloses, des dérivés de cellulose, de la poudre de lait, des oligosaccharides non hygroscopiques, du trisilicate de magnésium, du dioxyde de silicium et
b) le remplissage de la capsule contient au moins une matière miscible à l'eau, soluble dans l'eau, sensible à l'eau ou hydrophile, et facilement volatile.

2. Capsules de gélatine selon la revendication 1, caractérisées en ce que le remplissage de la capsule comprend une ou plusieurs matières du groupe des alcools inférieurs, des éthers, des esters, des alcanals, des alcanones, des polyols et des amines.

3. Capsules de gélatine selon la revendication 1, caractérisées en ce que le remplissage des capsules comprend une ou plusieurs matières du groupe des solutions de sucre aqueuses concentrées, des sirops d'amidon, des sirops d'amidon hydratés, des solutions de dérivés de sucre, et du miel.

4. Capsules de gélatine selon l'une des revendications 1 à 3, caractérisées en ce que l'enveloppe de la capsule est celle d'une capsule en gélatine molle.

5. Capsules en gélatine selon l'une des revendications 1 à 3, caractérisées en ce que l'enveloppe de la capsule est celle d'une capsule à mâcher molle.

6. Procédé de fabrication de capsules de gélatine selon l'une des revendications 1 à 5, caractérisé en ce que

a) il est ajouté à la décoction de gélatine, à titre d'addition une suspension ou une solution d'au moins 1% de poids d'un produit qui, sous la forme pure, peut absorber au moins 10% de son propre poids d'eau, sans subir de modifications extérieures, ce produit étant constitué par une ou plusieurs matières choisies dans le groupe des amidons, des dérivés d'amidon, des celluloses, des dérivés de cellulose, de la poudre de lait, des oligosaccharides non hygroscopiques, du trisilicate de magnésium, du dioxyde de silicium et

b) on utilise comme remplissage de la capsule au moins une matière miscible à l'eau, soluble dans l'eau, sensible à l'eau ou hydrophile et facilement volatile.

**Claims**

1. Gelatin capsules consisting of
a) a capsule sheath and
b) a capsule filling, the capsule sheath consisting of gelatin, optionally a plasticizer and further additives and the capsule filling containing active ingredients and/or dietary agents and optionally further additives and a liquid or a powder, characterized in that

a) the capsule sheath contains, as an additive, at least 1% by weight of an agent which in its pure state is capable of absorbing water in an amount of at least 10% of its own weight without undergoing any external change, said agent consisting of one or more substances from the group of the starches, starch derivatives, celluloses, cellulose derivatives, milk powder, non-hygroscopic oligosaccharides, magnesium trisilicate, silicon dioxide, and

b) the capsule filling contains at least one water miscible, water-soluble, water-sensitive or hydrophilic readily volatile substance.

2. The gelatin capsules according to claim 1, characterized in that the capsule filling contains one or more substances from the group of the lower alcohols, ethers, esters, alkanals, alkanones, polyols and amines.

3. The gelatin capsules according to claim 1, characterized in that the capsule filling contains one or more substances from the group of the concentrated aqueous sugar solutions, starch syrups, hydrogenated starch syrups, sugar derivative solutions, and honey.

4. The gelatin capsules according to any of claims 1 to 3, characterized in that the capsule sheath is that of a soft gelatin capsule.

5. The gelatin capsules according to any of claims 1 to 3, characterized in that the capsule sheath is that of a soft chewing capsule.

6. A process for the preparation of gelatin capsules according to any of claims 1 to 5, characterized in that

a) a suspension or solution of at least 1% by weight of an agent is added, as an additive, to the gelatin stock, which agent, in its pure state, is capable of absorbing water in an amount of at least 10% of its own weight without undergoing any external change, said agent consisting of one or more substances from the group of the starches, starch derivatives, celluloses, cellulose derivatives, milk powder, non-hygroscopic oligosaccharides, magnesium trisilicate, silicon dioxide, and

b) at least one water-miscible, water-soluble, watersensitive or hydrophilic readily volatile substance is employed as the capsule filling.